# EUROPEAN PATENT SPECIFICATION

(11) **EP 2 135 937 B1**
(45) Date of publication and mention of the grant of the patent: **27.09.2017**
(21) Application number: 08380179.5
(22) Date of filing: 20.06.2008
(51) Int. Cl.: C12M 1/00

(54) **Continuous process for the generation of high nutritional value and energy resources**
Kontinuierliches Verfahren zur Erzeugung von hohen Nährwerten und Energieressourcen
Procédé continu pour la génération de valeur nutritionnelle élevée et de ressources énergétiques

(43) Date of publication of application: 23.12.2009
(73) Proprietor: Stroïazzo-Mougin, Bernard A. J., 03560 El Campello (Alicante) (ES)
(72) Inventor: Stroïazzo-Mougin, Bernard A. J., 03560 El Campello (Alicante) (ES); Rodriguez Verdu, Eduardo, 03002 Alicante (ES); Chapuli Fernandez, Eloy, 3560 El Campello (Alicante) (ES)
(74) Representative: Oficina Ponti, SLP

(56) References cited:
- WO-A-91/05849
- WO-A-96/23865
- WO-A-98/45409
- WO-A-99/61577
- WO-A-2007/134141
- WO-A-2007/144440
- WO-A-2007/144441
- DE-A1- 10 315 750
- LI ET AL: "Effects of electromagnetic field on the batch cultivation and nutritional composition of Spirulina platensis in an air-lift photobioreactor" BIORESOURCE TECHNOLOGY, ELSEVIER, GB, vol. 98, no. 3, 1 February 2007 (2007-02-01), pages 700-705, XP005656285 ISSN: 0960-8524
- FACHINI ADRIANO ET AL: "Effects of zeolites on cultures of marine micro-algae - A brief review" ENVIRONMENTAL SCIENCE AND POLLUTION RESEARCH INTERNATIONAL, ECOMED, LANDSBERG, DE, vol. 13, no. 6, 1 October 2006 (2006-10-01), pages 414-417, XP009105875 ISSN: 0944-1344

## Description

### Object of the Invention

The object of the present invention is a continuous process for the generation of added value and energy resources.

### Backaround of the Invention

A huge amount of solar energy irradiates the surface of the earth, approximately about 15,000 times the current energy consumption. It is the largest energy source of our planet.

Nevertheless, the effectiveness of the concentration of energy per unit area at any point of the earth is very low, only about 1 to 5 kWh/m²/day. This low effectiveness of energy concentration limits the direct use of solar energy as a primary energy source.

An objective is to pass from this low energy density state to a high density state through a system for capturing sunlight and CO₂ to convert it into a continuous energy source using a magneto-hydro-photosynthetic catalyst, the latter being the actual system.

Based on this principle, application WO2007144440 is known which describes a photoconverter for a function similar to the present system, nevertheless, its horizontal and not vertical constitution, as well as other features, make it essentially different.

Global warming is currently a problem that is giving rise to the development of new technologies in different fields of the art in order to attempt eradicating or minimize the effects of said warming. The obligation of the economic areas to comply with the objectives imposed by the Kyoto protocol on the reduction of CO₂/SO₂ emissions and the emissions of other gases causing the so-called greenhouse effect is leading countries to search for alternative and renewable fuels to avoid possible tax penalties.

Although the production of solar and wind energy is increasing in some regions, these technologies are very expensive, they are not viable in all climatic areas and do not assure a continuous energy production. In these conditions, biofuels are intended to play an essential role as substitutes for fossil fuels, especially for transport and heating applications.

The production costs of biofuels from terrestrial plants, such as palm and rapeseed oil, have always been a reason for concern. Taking into account the low production indices per hectare, huge amounts of resources would be needed for a commercial production to be reached. Land and water are two scarce resources and it is preferable to use them for producing food, which are furthermore more profitable for farmers. Furthermore, intensive fertilization is a form of land and water pollution of the first order. Extensive single crop farming is also one of the main enemies of biodiversity.

A study of the University of California-Berkeley, Natural Resources Research Vol 14 No. 1 March 2005 pg. 65-72 shows that a terrestrial plant, such as sunflower, requires more energy for its transformation into a fuel than the energy capable of supplying as such, for example, for the production of 1,000 kg of sunflower fuel having a calorific value of 9,000,000 Kcal, 19 million Kcal have to be consumed, which corresponds to a CO₂ emission greater than that emitted by a fossil fuel. In other words, if the emission of a 135 hp (100 kW) car on a 100 km trip making use of a fossil fuel is 20 Kg of CO₂ when a sunflower-based fuel is used, the total combined emission would be 36 Kg of CO₂. However, in the case of a fuel based on phytoplankton fed with the CO2 of a power station, for example a thermal power station, the amount of CO2 emitted to the atmosphere is 10 kg. This is due to the fact that the CO₂ captured by the phytoplankton has generated in turn a power of 100 kW, therefore the balance of CO₂ emitted per kW produced is half. In addition, the present invention describes a system in which not all the end product is intended for the production of fuels, but also for the production of a high nutritional value product.

It should be emphasized that when the application of the end product is that of generating electric energy, there is no CO₂ emission to the atmosphere as such CO₂ is introduced again in the system for the production of phytoplankton.

In view of that discussed above, with a production of phytoplankton which allows substituting the use of fuels of a petroliferous origin, the advantages from the environmental point of view are evident. According to this, the great challenge lies in achieving a culture which allows massively producing this product. To that end, it is essential to suitably select the reactor to be used.

The reactors that are currently used, either on an industrial or experimental scale, can be classified into two large groups:
- Open reactors
- Closed reactors
   ∘ Horizontal
   ∘ Vertical

### Open reactors

They consist of the use of shallow tanks in which algae, water and nutrients circulate through the circuit by means of a motor-driven system of blades.

The great drawback represented by this system is that, since it is completely open, the algae are susceptible of being contaminated by any organism that is introduced into the tank. Furthermore, the conditions of the system, such as temperature, evaporation of water from the culture medium, supply of CO₂ and luminosity are more difficult to control, the photochemical efficiency being low (high cell densities are not reached); therefore the depth of the tank is of a few millimeters, thus requiring large areas to reach high productions. The group of these factors makes this system not be the most suitable one for the mass cultivation of phytoplankton species.

### Closed reactors

These reactors allow overcoming some of the main obstacles faced by an open system; they allow controlling the variables of the system (CO₂, luminosity, temperature...), there is no contamination, and they require smaller areas of land to reach the same production as in open reactors, especially in the event that the arrangement is vertical (greater volume per unit area). All of this makes the photochemical efficiency of the culture higher.

Due to that mentioned above, different authors have developed systems and processes in which photosynthetic phytoplankton species are cultivated which are exposed to light to generate a growth and multiplication thereof in order to finally obtain a usable product (after several modification steps), as is the case of PCT applications WO2007025145A2, WO2006/020177, WO 03/094598 and WO2007144441. However all these systems have the problem that they do not work in a clearly continuous manner, do not have a suitable photochemical efficiency, do not have a recirculation of part of their own obtained products, are laborious systems which in some cases are susceptible to contamination, use solar energy in a two-dimensional manner (they do not take advantage of the verticality of the system of the present invention), and in short, have high associated costs (cleaning, disinfection, evaporation of culture water, are energetically deficient [pumping, extraction...]...), and which indirectly generate contamination. Furthermore, systems or processes such as that described in WO2007025145A2 work according to a horizontal arrangement (they use stirring by means of rollers), the resulting products are not reused (as is done in the present invention with the NOx passing through biofilters to be transformed into nitrates and nitrites which are used as a supply of nutrients to the culture medium). These systems or processes obtain amounts of fuels (about 6000 gallons) lower than the system of the present invention.

Document DE 10315750A1 discloses a system for producing biomass such as phytoplankton using a photobioreactor.

In addition, the process described herein has the additional advantage that in the production step a magnetic field is applied which causes an acceleration of said step and, therefore, a substantial increase of the amount of obtained product.

In this same sense, the present invention relates to a novel continuous process for obtaining energy resources by means of a system for capturing sunlight and CO₂ for the reconversion thereof into a continuous energy source using catalysts. To minimize direct or indirect contamination, the process, which will be described below, uses nutrients such as atmospheric CO₂, carbon and nitrogen sources from different industrial sectors. By means of the present invention a total or partial recycling (depending on the application) of the CO₂ is achieved and therefore the net emission can be "zero", since the CO₂ generated can return to the system, thus nourishing the phytoplankton. Furthermore, for the purpose of minimizing water consumption, the water is reused after the separation step which will be described in more detail below.

Furthermore, the process has the advantage that the oxygen generated in one of its steps is reused as a raw material for a subsequent transformation of the obtained product.

Another advantage is that up until now there is no process in which a mass culture of photosynthetic strains is carried out as is done in the present invention.

Thus a substantial improvement of the processes that are already known in the state of the art is achieved, either in efficiencies (energetic-economic and productive efficiencies) or in minimization of environmental impact. It is therefore a sustainable process both from the energetic and ecological point of view.

### Description of the Invention

To palliate the problems mentioned above, the continuous process for the generation of added value and energy resources, object of the present invention, is as set forth in the set of claims. Said process uses a system comprising at least:
(i) first production means for producing phytoplankton culture, wherein said first means in turn comprise at least:
   (a) a plurality of production modules for producing phytoplankton culture that are vertically placed, fed with CO2 and interconnected to one another in the form of a closed circuit comprising a recirculation pump for recirculating the phytoplankton culture and a plurality of generation means for generating a magnetic field located in the recirculation pipes, such that either a continuous or alternating excitation magnetic field is generated in said area;
   (b) a container structure for containing the production modules, such that the modules-structure assembly is stackable and has dimensions such that its transport in a standard transport container is allowed;
   (c) natural (sunlight, sunlight concentrators-distributors) and/or artificial (fluorescent tubes, LEDs) illumination means, wherein said illumination means can be internal or external with regard to the production modules for producing culture;

Working in parallel is thus achieved between the production modules, assuring the same amount of fluid in a margin comprised between 10% and 15% of flow variation and one and the same load for all the modules. The system also allows an amount of processing of the phytoplankton culture of up to 20000 liters/hour, preferably 5000 liters/hour.

The production modules for producing phytoplankton culture comprise a plurality of columns connected to one other, and wherein said columns in turn comprise:
(i) a tube assembly with a maximum height of 10 meters and a preferred height between 2 and 2.25 meters, containing the phytoplankton culture and said tube assembly comprising at least:
   (a) a first external tube comprising, in turn, at least one of the following layers:
      - a first outer layer of ultraviolet treatment;
      - a second intermediate layer of a material such that it is resistant to the pressure of the volume contained; and
      - a third antiadherent inner layer, in contact with the contained fluid;
   (b) a second internal tube with regard to the first tube, said second tube comprising at least one of the following layers:
      - a first antiadherent outer layer, in contact with the contained fluid;
      - a second intermediate layer of a material resistant to the pressure of the volume contained in the first tube; and
      - a third inner layer of ultraviolet treatment;
   (c) upper and lower assembly closure elements; and
   (d) inter-column connection elements, comprising a fluid connector between columns and a plurality of auxiliary fluid circulation elements;
   (e) cleaning means comprising at least two concentric rings, a first outer ring and a second inner ring and wherein both are joined by means of a plurality of radii, and a peripheral element which cleans by friction the walls with which it comes into contact without damaging them;
   (f) a cooling and/or refracting element (a third outer tube with regard to the first external tube which would act as a sleeve), comprising in turn refracting subelements and/or a cooling and/or refracting liquid therein;
   (g) a medium containing zeolites;
   such that the second internal tube is inserted in the first external tube, the assembly being closed at its ends, the inter-column connection elements being located both at its upper part and at its lower part; and
(ii) feed and turbulence generation means for generating turbulences of a fluid in gaseous state and for the feeding thereof, this fluid in gaseous state being at least one selected from:
   (a) air;
   (b) N₂;
   (c) CO₂;
   (d) industrial emission exhaust gases; or
   (e) a combination thereof.

The production modules for producing phytoplankton culture comprise four production columns for producing phytoplankton culture connected in parallel and/or in series.

The verticality of the production modules provides clear advantages compared to the traditional horizontal placement described in the state of the art, thus, for example, the capacity of discharging the oxygen which is generated in the culture is considerably improved. Other inherent advantages are: a considerable improvement of the production, since the ratio of the volume per unit area increases; the necessary pumping requires a lower electric consumption, it being possible to stop the recirculation pump to prevent unnecessary energy consumptions during certain periods of the production step.

The modularity of the system is another considerable advantage, especially in actions of preventive maintenance and/or contamination of the phytoplankton culture, wherein only is necessary to treat the affected module and the rest of the system is not affected.
(ii) The system likewise comprises second separation means connected to the outlet of the first production means, such that the water present in the latter is eliminated; and wherein said separation means comprise at least:
   (a) phytoplankton culture pre-concentration means, connected to the outlet of the first production means;
   (b) pre-concentrated product concentration means, connected to the outlet of the pre-concentration means; and
   (c) thermal drying means, connected to the outlet of the concentration means;
(iii) The system comprises third transformation means for transforming the dry product obtained in the second means, such that an energy resource and/or high nutritional value product is obtained, wherein said third means comprise means selected from:
   (a) gasification means, such that a gas suitable for its use as a fuel is obtained; or
   (b) pyrolysis transformation means such that a product is obtained selected from:
      - a fuel oil;
      - a liquid mixture of organic compounds which, after a refining process, allows obtaining different fractions with physicochemical characteristics similar to those obtained from petroleum.
      - an ester which, in combination with fatty acid dissolution and extraction means and esterification/transesterification means, allows obtaining a diesel compound;
   (c) nutritional product extraction means;
   (d) a combination of the above.

The pre-concentration means of the second separation means comprise at least one of the following systems or means:
- a submerged membrane filtration system (MBR);
- a tangential filtration system;
- flocculation and/or coagulation means;
- electrocoagulation means;
- an ultrasound system;
- mechanical vapor recompression (MVR) means;
- centrifugation means;
- decantation means; or
- a combination of the above;
such that up to 99% of the water present in the production means is eliminated.

The concentration means comprise at least one system selected from:
- a mechanical vapor recompression (MVR) system;
- pre-concentrated element decantation means;
- centrifugation means;
- pressing means; or
- a combination of the above;
such that a concentrate with a relative humidity comprised between 40% and 90%, preferably between 65 and 70%, is obtained.

The drying means comprise, at least:
(i) thermal drying means for drying by hot air, such that the concentrated product can be dried by means of the insertion of hot air with a temperature not less than 75°;
(ii) lyophilization means;
(iii) multiple effect evaporators.

The invention thus described, including the vertical arrangement of the production modules, allows obtaining the following advantages:
a) containing more volume of culture per unit area, therefore more productivity per unit area;
b) that the means for generating turbulence have an effect of cleaning by friction, both for the inner wall of the outer tube and for the outer wall of the inner tube;
c) an elimination of the oxygen generated during the production step;
d) increasing the three-dimensional capture of light;
e) elimination of the shadow effect
f) lower electric consumption
g) exhaust gas assimilation capacity

### Brief Description of the Drawings

A series of drawings is very briefly described below which aid in better understanding the invention and which are expressly related to an embodiment of said invention which is set forth as a non-limiting example thereof.

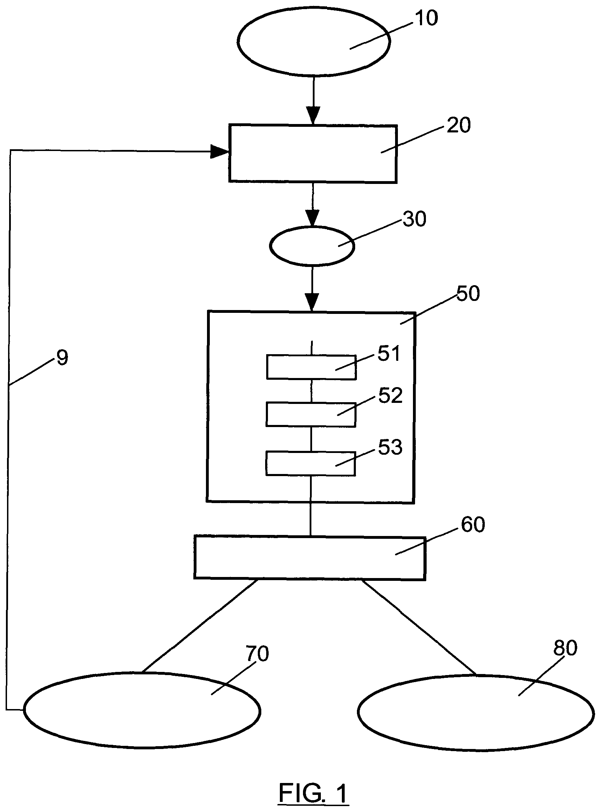

Figure 1 shows a block diagram of the continuous system for the generation of high nutritional value and energy resources, object of the present invention patent.

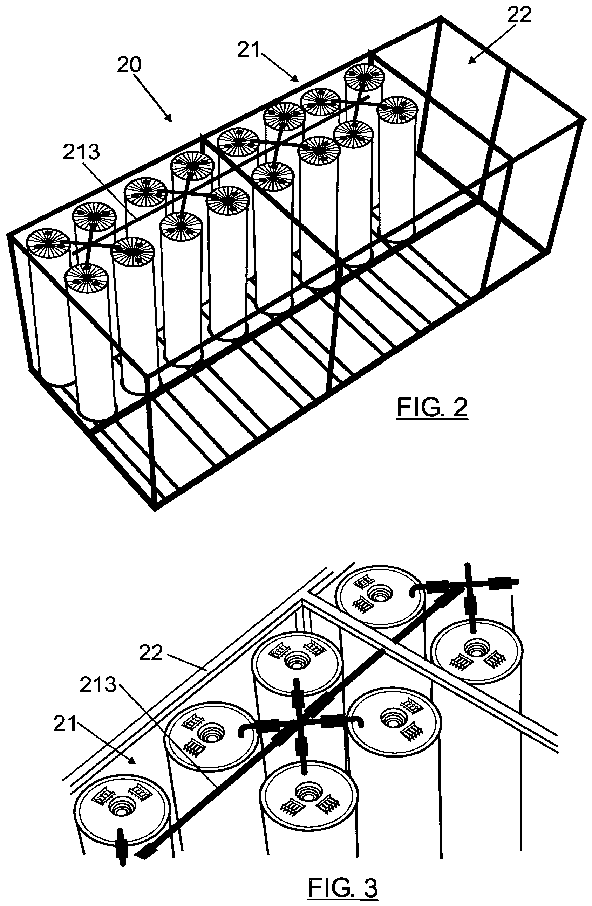

Figure 2 shows an upper perspective view of the production means for producing phytoplankton culture, an integral part of the system object of the present invention patent.
Figure 3 shows a detailed view of the joining of the production modules for producing phytoplankton culture, an integral part of the system object of the present invention patent.

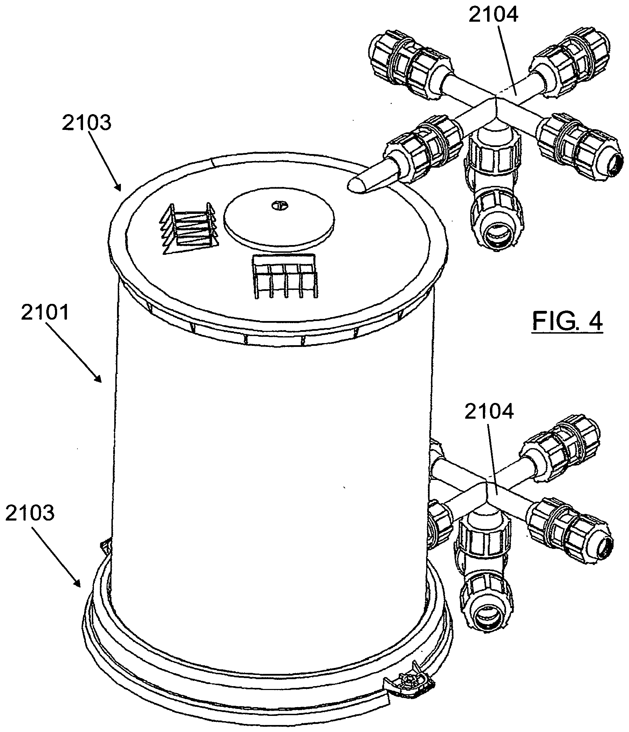

Figure 4 shows a detailed view of a production module.

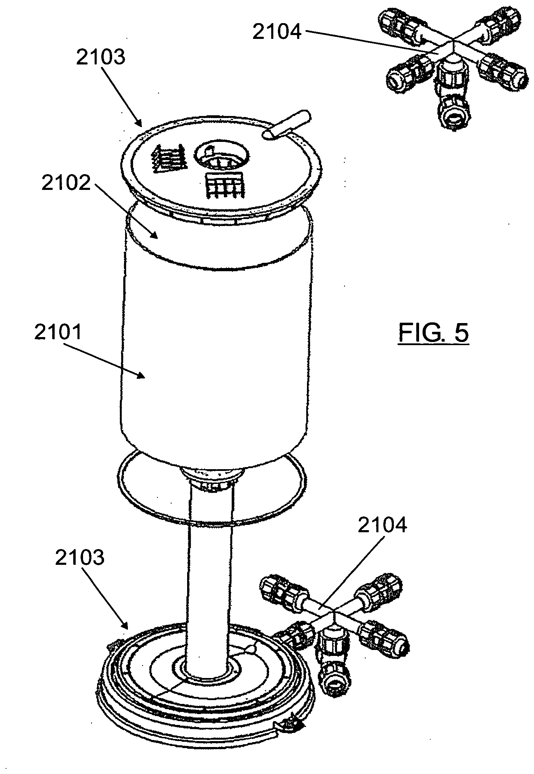

Figure 5 shows an exploded view of the production module shown in Figure 4.

### Preferred Embodiment of the Invention

As can be seen in the attached figures, the continuous process for the generation of high nutritional value and energy resources, in its preferred embodiment, uses a system comprising at least:
(i) first production means (20) for producing phytoplankton culture (10), wherein said first means (20) in turn comprise at least:
   (a) a plurality of production modules (21) for producing phytoplankton culture that are vertically placed, fed with CO₂ and interconnected to one another (213) in the form of a closed circuit, comprising a recirculation pump for recirculating the phytoplankton culture and a plurality of generation means for generating a magnetic field located in the recirculation pipes (213), such that either a continuous or alternating excitation magnetic field is generated in said area;
   (b) a container structure (22) for containing the production modules, such that the modules-structure assembly is stackable and has dimensions such that its transport in a standard transport container is allowed;
   (c) natural (sunlight, sunlight concentrators-distributors) and/or artificial (fluorescent tubes, LEDs) illumination means, wherein said illumination means can be internal or external with regard to the production modules for producing culture;

Working in parallel is thus achieved between the production modules (21), assuring the same amount of fluid in a margin comprised between 10% and 15% of flow variation and one and the same load for all the modules (21). The system also allows an amount of processing of the phytoplankton culture of up to 20000 liters/hour, preferably 5000 liters/hour.

The production modules (21) for producing phytoplankton culture (10) comprise a plurality of columns connected to one another, and wherein said columns in turn comprise:
(i) a tube assembly (210) with a maximum height of 10 meters and a preferred height between 2 and 2.25 meters, containing the phytoplankton culture and said tube assembly (210) comprising at least:
   (a) a first external tube (2101) comprising, in turn, at least one of the following layers:
      - a first outer layer of ultraviolet treatment;
      - a second intermediate layer of a material such that it is resistant to the pressure of the volume contained; and
      - a third antiadherent inner layer, in contact with the contained fluid;
   (b) a second internal tube (2102) with regard to the first tube (2101), said second tube comprising at least one of the following layers:
      - a first antiadherent outer layer, in contact with the contained fluid;
      - a second intermediate layer of a material resistant to the pressure of the volume contained in the first tube; and
      - a third inner layer of ultraviolet treatment;
   (c) upper and lower assembly closure elements (2103); and
   (d) inter-column connection elements (2104), comprising a fluid connector between columns and a plurality of auxiliary fluid circulation elements;
   (e) cleaning means comprising at least two concentric rings, a first outer ring and a second inner ring and wherein both are joined by means of a plurality of radii, and a peripheral element which cleans by friction the walls with which it comes into contact without damaging them;
   (f) a cooling and/or refracting element (a third outer tube with regard to the first external tube which would act as a sleeve), comprising in turn refracting subelements and/or a cooling and/or refracting liquid therein;
   (g) a medium containing zeolites; such that the second internal tube (2102) is inserted in the first external tube (2101), the assembly being closed at its ends, the inter-column connection elements (2104) being located both at its upper part and at its lower part; and
(ii) feed and turbulence generation means for generating turbulences of a fluid in gaseous state and for the feeding thereof, this fluid in gaseous state being at least one selected from:
   (a) air;
   (b) N₂;
   (c) CO₂;
   (d) industrial emission exhaust gases; or
   (e) a combination thereof.

The production modules (21) for producing phytoplankton culture (10) comprise four production columns (210) for producing phytoplankton culture connected in parallel and/or in series.

The system likewise comprises second separation means (50) connected to the outlet of the first production means (20), such that the water present in the latter is eliminated after an emptying step (30); and wherein said separation means comprise at least:
(a) phytoplankton culture pre-concentration means (51), connected to the outlet of the first production means (20);
(b) pre-concentrated product concentration means (52), connected to the outlet of the pre-concentration means (51); and
(c) thermal drying means (53), connected to the outlet of the concentration means (52);

The system comprises third transformation means (60) for transforming the dry product obtained in the second means (50), such that an energy resource (70) and/or high nutritional value product (60) is obtained, wherein said third means comprise means selected from:
(a) gasification means, such that a gas suitable for its use as a fuel is obtained; or
(b) pyrolysis transformation means such that a product is obtained selected from:
   - a fuel oil;
   - a liquid mixture of organic compounds which, after a refining process, allows obtaining different fractions with physicochemical characteristics similar to those obtained from petroleum;
   - an ester which, in combination with fatty acid dissolution and extraction means and esterification/transesterification means, allows obtaining a diesel compound;
(c) nutritional product extraction means;
(d) a combination of the above.

The pre-concentration means of the second separation means comprise at least one of the following systems or means:
- a submerged membrane filtration system (MBR);
- a tangential filtration system;
- flocculation and/or coagulation means;
- electrocoagulation means;
- an ultrasound system;
- mechanical vapor recompression (MVR) means;
- centrifugation means;
- decantation means; or
- a combination of the above;
such that up to 99% of the water present in the production means is eliminated.

The concentration means comprise at least one system selected from:
- a mechanical vapor recompression (MVR) system;
- pre-concentrated element decantation means;
- centrifugation means;
- pressing means; or
- a combination of the above;
such that a concentrate with a relative humidity comprised between 40% and 90%, preferably between 65 and 70%, is obtained.

The drying means comprise, at least:
(i) thermal drying means for drying by hot air, such that the concentrated product can be dried by means of the insertion of hot air with a temperature not less than 75°;
(ii) lyophilization means;
(iii) multiple effect evaporators.

In a practical embodiment, the CO₂ used for feeding the phytoplankton culture has its origin in a feedback from the transformation means.

In the same way, the system in another practical embodiment incorporates wind generators, such that said wind generators generate the actual electricity for the use of the system, an excess energy accumulation being generated in the phytoplankton culture itself.

Finally, in a last embodiment the system is installed in the sea, by means of floats and partially submerged.

## Claims

1. A continuous process for the generation of high nutritional value and energy resources, using a system that comprises at least:
(i) first production means (20) for producing phytoplankton culture (10), wherein said first means (20) in turn comprise at least:
(a) a plurality of production modules (21) for producing phytoplankton culture that are vertically placed, fed with CO₂ and interconnected to one another (213) in the form of a closed circuit, comprising a recirculation pump for recirculating the phytoplankton culture and generation means for generating a magnetic field located in the recirculation pipes (213), such that an excitation magnetic field for exciting the culture is generated in said area;
(b) a container structure (22) for containing the production modules, such that the modules - structure assembly is stackable and has dimensions such that its transport in a standard transport container is allowed;
(c) a plurality of illumination means;
(ii) second separation means (50) connected to the outlet of the first production means (20), after an emptying step (30), such that the water present in the culture produced in the first means (20) is eliminated; and wherein said separation means (50) comprise at least:
(a) pre-concentration means (51), connected to the inlet of culture from the first production means (20);
(b) pre-concentrated product concentration means (52), connected to the outlet of the pre-concentration means (51); and
(c) thermal concentrated product drying means (53), connected to the outlet of the concentration means (52);
(iii) third transformation means (60) for transforming the dried product obtained in the second means (50), such that an energy resource (70) and/or high nutritional value product (80) is obtained, wherein said third means (60) comprise means selected from:
(a) gasification means, such that a gas suitable for its use as a fuel is obtained; or
(b) pyrolysis transformation means such that a product is obtained selected from:
- an oil;
- a liquid mixture of organic products which, after a standard refining process, allows obtaining different fractions with physicochemical characteristics similar to those obtained from petroleum;
- an ester which, in combination with fatty acid dissolution and extraction means and esterification/transesterification means, allows obtaining a diesel compound;
(c) nutritional product extraction means;
(d) a combination thereof,
**characterised in that** the production modules (21) work in parallel with a culture flow variation in a margin comprised between 10% and 15%, and the culture load is the same for all the modules (21).

2. The continuous process according to claim 1, **characterized in that** the production modules (21) comprise a plurality of production columns connected to one another, and wherein said columns in turn comprise:
(i) a tube assembly (210) containing the phytoplankton culture, said tube assembly (210) comprising at least:
(a) a first external tube (2101) comprising at least one of the following layers:
- a first outer layer of ultraviolet treatment;
- a second intermediate layer of a material resistant to the pressure of the volume contained; and
- a third antiadherent inner layer, in contact with the contained fluid;
(b) a second internal tube (2102) with regard to the first tube (2101), comprising at least one of the following layers:
- a first antiadherent outer layer, in contact with the contained fluid;
- a second intermediate layer of a material resistant to the pressure of the volume contained in the first tube; and
- a third inner layer of ultraviolet treatment;
(c) upper and lower assembly closure elements (2103); and
(d) inter-column connection elements (2104), comprising a fluid connector between columns and a plurality of auxiliary fluid circulation elements;
(e) cleaning means comprising at least two concentric rings, a first outer ring and a second inner ring and wherein both are joined by means of a plurality of radii, and a peripheral element which cleans by friction the walls with which it comes into contact without damaging them;
(f) a cooling and/or refracting element, consisting of a third outer tube with regard to the first external tube such that it acts as a sleeve, comprising in turn refracting sub-elements and/or a cooling and/or refracting liquid therein;
(g) a medium containing zeolites;
such that the second internal tube (2102) is inserted in the first external tube (2101), the assembly being closed at its ends, the inter-column connection elements (2104) being located both at its upper part and at its lower part; and
(ii) feed and turbulence generation means for generating turbulences of a fluid in gaseous state and for the feeding thereof, this fluid in gaseous state being at least one selected from:
(a) air;
(b) N₂;
(c) CO₂;
(d) industrial emission exhaust gases; or
(e) a combination thereof.

3. The continuous process according to claim 1, **characterized in that** the excitation magnetic field is continuous.

4. The continuous process according to claim 1, **characterized in that** the excitation magnetic field is alternating.

5. The continuous process according to claim 1, **characterized in that** the pre-concentration means (51) of the second separation means (50) comprise at least one of the following systems:
- a submerged membrane filtration system (MBR);
- a tangential filtration system;
- flocculation and/or coagulation means;
- electrocoagulation means;
- an ultrasound system;
- mechanical vapor recompression (MVR) means;
- centrifugation means;
- decantation means; or
- a combination of the above;
such that 99% of the water present in the culture is eliminated.

6. The continuous process according to claim 1, **characterized in that** the concentration means (52) comprise at least one system selected from:
- a mechanical vapor recompression (MVR) system;
- pre-concentrated element decantation means ;
- centrifugation means;
- pressing means; or
- a combination of the above;
such that a concentrate with a relative humidity comprised between 40% and 90%, preferably between 65 and 70%, is obtained.

7. The continuous process according to the claim 1, **characterized in that** the drying means (53) comprise at least:
(i) thermal drying means for drying by hot air, such that the concentrated product can be dried by means of the insertion of hot air with a temperature not less than 75°C;
(ii) lyophilization means;
(iii) multiple effect evaporators.

8. The continuous process according to claims 1 and 2, **characterized in that** the CO₂ used for feeding the phytoplankton culture has its origin in a feedback from the transformation means.

9. The continuous process according to any of the previous claims, **characterized in that** the system incorporates wind generators, such that said wind generators generate the actual electricity for the use of the system, an excess energy accumulation being generated in the phytoplankton culture itself.

10. The continuous process according to any of the previous claims, **characterized in that** the system is installed in the sea, by means of floats and partially submerged.

11. The continuous process according to the claim 1, **characterized in that** the illumination means are either internal or external with regard to the production modules for producing culture and comprise natural and/or artificial illumination means.

12. The continuous process according to claim 1, wherein the phytoplankton culture is processed at a rate of up to 20,000 litres/hour.

13. The continuous process according to claim 12, wherein the phytoplankton culture is processed at a rate of up to 5,000 litres/hour.

## Patentansprüche

1. Ein kontinuierlicher Prozess für die Erzeugung von Ressourcen mit hohem Nährwert und Energieressourcen unter Verwendung eines Systems, das zumindest folgende Merkmale aufweist:
(i) eine erste Produktionseinrichtung (20) zum Erzeugen einer Phytoplanktonkultur (10), wobei die erste Einrichtung (20) wiederum zumindest folgende Merkmale aufweist:
(a) eine Mehrzahl von Produktionsmodulen (21) zum Erzeugen einer Phytoplanktonkultur, die vertikal angeordnet sind, mit CO₂ gespeist werden und miteinander verbunden sind (213) in der Form eines geschlossenen Kreislaufs, der eine Umwälzungspumpe zum Umwälzen der Phytoplanktonkultur und eine Erzeugungseinrichtung zum Erzeugen eines Magnetfelds aufweist, die in den Umwälzungsrohren (213) angeordnet ist, so dass ein Erregungsmagnetfeld zum Erregen der Kultur in dem Bereich erzeugt wird;
(b) eine Behälterstruktur (22) zum Aufnehmen der Produktionsmodule, so dass die Module-Strukturanordnung stapelbar ist und Abmessungen aufweist, die deren Transport in einem Standardtransportbehälter ermöglichen;
(c) eine Mehrzahl von Beleuchtungseinrichtungen;
(ii) eine zweite Trennungseinrichtung (50), die mit dem Auslass der ersten Produktionseinrichtung (20) verbunden ist, nach einem Entleerungsschritt (30), so dass das Wasser, das in der Kultur vorhanden ist, die in der ersten Einrichtung (20) produziert wird, entfernt wird; und wobei die Trennungseinrichtung (50) zumindest folgende Merkmale aufweist:
(a) eine Vorkonzentrierungseinrichtung (51), die mit dem Einlass der Kultur von der ersten Produktionseinrichtung (20) verbunden ist;
(b) eine Vorkonzentriertes-Produkt-Konzentrierungseinrichtung (52), die mit dem Auslass der Vorkonzentrierungseinrichtung (51) verbunden ist; und
(c) eine Thermisch-Konzentriertes-Produkt-Trockeneinrichtung (53), die mit dem Auslass der Konzentrierungseinrichtung (52) verbunden ist;
(iii) eine dritte Transformationseinrichtung (60) zum Transformieren des getrockneten Produkts, das in der zweiten Einrichtung (50) erhalten wird, so dass eine Energieressource (70) und/oder ein Produkt mit hohem Nährwert (80) erhalten wird, wobei die dritte Einrichtung (60) eine Einrichtung aufweist, die aus Folgendem ausgewählt ist:
(a) eine Vergasungseinrichtung, so dass ein Gas, das für die Nutzung als Kraftstoff geeignet ist, erhalten wird; oder
(b) eine Pyrolysetransformationseinrichtung, so dass ein Produkt erhalten wird, das aus Folgendem ausgewählt ist:
- ein Öl;
- eine Flüssigmischung aus organischen Produkten, die nach einem Standardverfeinerungsprozess das Erhalten unterschiedlicher Anteile mit physikochemischen Charakteristika ermöglicht, die ähnlich denjenigen sind, die von Erdöl erhalten werden;
- ein Ester, das in Kombination mit einer Fettsäure-Auflösungs- und Extraktionseinrichtung und einer Veresterungs/Umesterungseinrichtung das Erhalten eines Dieselgemischs ermöglicht;
(c) eine Nahrungsmittelprodukt-Extraktionseinrichtung;
(d) eine Kombination derselben,
**dadurch gekennzeichnet, dass** die Produktionsmodule (21) parallel arbeiten mit einer Kulturflussschwankung innerhalb einer Spanne von 10 % bis 15 %, und die Kulturlast für alle Module (21) die gleiche ist.

2. Der kontinuierliche Prozess gemäß Anspruch 1, **dadurch gekennzeichnet, dass** die Produktionsmodule (21) eine Mehrzahl von Produktionssäulen aufweisen, die miteinander verbunden sind, und wobei die Säulen wiederum folgende Merkmale aufweisen:
(i) eine Röhrenanordnung (210), die die Phytoplanktonkultur enthält, wobei die Röhrenanordnung (210) zumindest folgende Merkmale aufweist:
(a) eine erste äußere Röhre (2101), die zumindest eine der folgenden Schichten aufweist:
- ein erste äußere Schicht mit Ultraviolett-Behandlung;
- eine zweite Zwischenschicht eines Materials, das widerstandsfähig ist gegenüber dem Druck des enthaltenen Volumens; und
- eine dritte innere Antihaftschicht in Kontakt mit dem enthaltenen Fluid;
(b) eine zweite innere Röhre (2102) mit Bezug auf die erste Röhre (2101), die zumindest eine der folgenden Schichten aufweist:
- eine erste äußere Antihaftschicht in Kontakt mit dem enthaltenen Fluid;
- eine zweite Zwischenschicht eines Materials, das widerstandsfähig ist gegenüber dem Druck des Volumens, das in der ersten Röhre enthalten ist; und
- eine dritte innere Schicht mit Ultraviolett-Behandlung;
(c) ein oberes und unteres Anordnungsverschlusselement (2103); und
(d) Zwischensäulenverbindungselemente (2104), die einen Fluidverbinder zwischen Säulen und eine Mehrzahl von Nebenfluidzirkulationselementen aufweisen;
(e) eine Reinigungseinrichtung, die zumindest zwei konzentrische Ringe, einen ersten äußeren Ring und einen zweiten inneren Ring, die beide durch eine Mehrzahl von Speichen verbunden sind, und ein Umfangselement aufweist, das die Wände, mit denen dasselbe in Kontakt kommt, durch Reibung reinigt ohne dieselben zu beschädigen;
(f) ein Kühl- und/oder Brechungselement, das aus einer dritten äußeren Röhre mit Bezug auf die erste äußere Röhre besteht, so dass dieselbe als eine Hülse wirkt, die wiederum brechende Teilelemente und/oder eine kühlende und/oder brechende Flüssigkeit darin aufweist;
(g) ein Medium, das Zeolithe enthält;
so dass die zweite innere Röhre (2102) in die erste äußere Röhre (2101) eingefügt ist, wobei die Anordnung an ihren Enden geschlossen ist, die Zwischensäulenverbindungselemente (2104) sowohl an dem oberen Teil als auch dem unteren Teil derselben angeordnet sind; und
(ii) eine Zuführungs- und Turbulenzerzeugungseinrichtung zum Erzeugen von Turbulenzen eines Fluids in einem gasförmigen Zustand und für die Zuführung desselben, wobei dieses Fluid in einem gasförmigen Zustand zumindest eines ist, das aus Folgenden ausgewählt ist:
(a) Luft;
(b) N₂;
(c) CO₂;
(d) Industrieemissionsabgase; oder
(e) eine Kombination derselben.

3. Der kontinuierliche Prozess gemäß Anspruch 1, **dadurch gekennzeichnet, dass** das Erregungsmagnetfeld kontinuierlich ist.

4. Der kontinuierliche Prozess gemäß Anspruch 1, **dadurch gekennzeichnet, dass** das Erregungsmagnetfeld wechselnd ist.

5. Der kontinuierliche Prozess gemäß Anspruch 1, **dadurch gekennzeichnet, dass** die Vorkonzentrierungseinrichtung (51) der zweiten Trennungseinrichtung (50) zumindest eines der folgenden Systeme aufweist:
- ein eingetauchtes Membranfiltrationssystem (MBR);
- ein Tangentialfiltrationssystem;
- eine Flockungs-und/oder Koagulationseinrichtung;
- eine Elektrokoagulationseinrichtung;
- ein Ultraschallsystem;
- eine Mechanische-Dampfrekompressions(MVR)-Einrichtung;
- eine Zentrifugationseinrichtung;
- eine Dekantierungseinrichtung; oder
- eine Kombination der obigen;
so dass 99 % des Wasser, das in der Kultur vorhanden ist, entfernt wird.

6. Der kontinuierliche Prozess gemäß Anspruch 1, **dadurch gekennzeichnet, dass** die Konzentrationseinrichtung (52) zumindest ein System aufweist, das aus Folgendem ausgewählt ist:
- ein Mechanische-Dampfrekompressions(MVR)-System;
- eine Vorkonzentriertes-Element-Dekantierungseinrichtung;
- eine Zentrifugationseinrichtung;
- eine Presseinrichtung; oder
- eine Kombination der obigen;
so dass ein Konzentrat mit einer relativen Feuchtigkeit, die zwischen 40 % und 90 %, vorzugsweise zwischen 65 % und 70 % liegt, erhalten wird.

7. Der kontinuierliche Prozess gemäß Anspruch 1, **dadurch gekennzeichnet, dass** die Trockeneinrichtung (53) zumindest folgende Merkmale aufweist:
(i) eine thermische Trockeneinrichtung zum Trocknen durch heiße Luft, so dass das konzentrierte Produkt getrocknet werden kann durch die Einführung heißer Luft mit einer Temperatur von nicht weniger als 75°C;
(ii) eine Gefriertrocknungseinrichtung;
(iii) ein Mehrfacheffektverdampfer.

8. Der kontinuierliche Prozess gemäß Anspruch 1 und 2, **dadurch gekennzeichnet, dass** das CO₂, das zum Speisen der Phytoplanktonkultur verwendet wird, seinen Ursprung in einer Rückkopplung von der Transformationseinrichtung hat.

9. Der kontinuierliche Prozess gemäß einem der vorhergehenden Ansprüche, **dadurch gekennzeichnet, dass** das System Windgeneratoren umfasst, so dass die Windgeneratoren die tatsächliche Elektrizität für die Nutzung des Systems erzeugen, wobei überschüssige Energieansammlung in der Phytoplanktonkultur selbst erzeugt wird.

10. Der kontinuierliche Prozess gemäß einem der vorherigen Ansprüche, **dadurch gekennzeichnet, dass** das System durch Schwimmer und teilweise unter Wasser im Meer installiert ist.

11. Der kontinuierliche Prozess gemäß Anspruch 1, **dadurch gekennzeichnet, dass** die Beleuchtungseinrichtungen mit Bezug auf die Produktionsmodule zum Erzeugen der Kultur entweder intern oder extern sind und natürliche und/oder künstliche Beleuchtungseinrichtungen aufweisen.

12. Der kontinuierliche Prozess gemäß Anspruch 1, bei dem die Phytoplanktonkultur bei einer Geschwindigkeit von bis zu 20.000 Liter/Stunde verarbeitet wird.

13. Der kontinuierliche Prozess gemäß Anspruch 12, bei dem die Phytoplanktonkultur bei einer Geschwindigkeit von bis zu 5.000 Liter/Stunde verarbeitet wird.

## Revendications

1. Procédé continu pour générer des ressources à haute valeur nutritionnelle et riches en énergie, utilisant un système comprenant au moins :
(i) des premiers moyens de production (20) pour produire une culture de phytoplanctons (10), dans lesquels lesdits premiers moyens (20) comprennent à leur tour au moins :
(a) une pluralité de modules de production (21) pour produire une culture de phytoplanctons placés de manière verticale, alimentés en CO₂ et interconnectés les uns aux autres (213) sous la forme d'un circuit fermé, comprenant une pompe de recirculation pour faire à nouveau circuler la culture de phytoplanctons et des moyens de génération pour générer un champ magnétique situé dans les tuyaux de recirculation (213), de telle sorte qu'un champ d'excitation magnétique pour exciter la culture est généré dans ladite zone ;
(b) une structure récipient (22) pour contenir les modules de production, de telle sorte que l'ensemble modules - structure soit empilable et ayant des dimensions telles qu'elles permettent son transport dans un conteneur de transport standard ;
(c) une pluralité de moyens d'éclairage ;
(ii) des seconds moyens de séparation (50) connectés à la sortie des premiers moyens de production (20), après une étape de vidage (30), de telle sorte que l'eau présente dans la culture produite dans les premiers moyens (20) soit éliminée ; et dans lesquels lesdits moyens de séparation (50) comprennent au moins :
(a) des moyens de pré-concentration (51), connectés à l'entrée de culture des premiers moyens de production (20) ;
(b) des moyens de concentration des produits pré-concentrés (52), connectés à la sortie des moyens de pré-concentration (51) ; et
(c) des moyens thermiques de séchage de produits concentrés (53), connectés à la sortie des moyens de concentration (52);
(iii) troisième moyen de transformation (60) pour transformer le produit séché obtenu dans les seconds moyens (50), de telle sorte qu'une ressource d'énergie (70) et/ou un produit à haute valeur nutritionnelle (80) soit obtenu, dans lesquels lesdits troisièmes moyens (60) comprennent des moyens sélectionnés parmi :
(a) des moyens de gazéification, de telle sorte qu'un gaz adapté à son utilisation en tant que carburant soit obtenu ; ou
(b) des moyens de transformation par pyrolyse de telle sorte qu'un produit soit obtenu, sélectionné parmi :
- une huile ;
- un mélange liquide de produits organiques qui, après un processus de raffinement standard, permet d'obtenir différentes fractions de caractéristiques physico-chimiques semblables à celles obtenues du pétrole ;
- un ester qui, en combinaison avec la dissolution d'acides gras et les moyens d'extraction et les moyens d'estérification/transestérification, permet d'obtenir un composé diesel ;
(c) des moyens d'extraction de produit nutritionnel ;
(d) une combinaison de ceux-ci,
**caractérisé en ce que** les modules de production (21) fonctionnent parallèlement à une variation de débit de culture dans une marge comprise entre 10% et 15% et **en ce que** la charge de culture est la même pour tous les modules (21).

2. Procédé continu selon la revendication 1, **caractérisé en ce que** les modules de production (21) comprennent une pluralité de colonnes de production connectées les unes aux autres, et dans lequel lesdites colonnes comprennent elles-mémes :
(i) un ensemble de tubes (210) contenant la culture de phytoplanctons, ledit ensemble de tubes (210) comprenant au moins :
(a) un premier tube extérieur (2101) comprenant au moins l'une des couches suivantes :
- une première couche extérieure à traitement ultraviolet ;
- une seconde couche intermédiaire d'un matériau résistant à la pression du volume contenu ; et
- une troisième couche intérieure antiadhésive, en contact avec le liquide contenu ;
(b) un second tube intérieur (2102) par rapport au premier tube (2101), comprenant au moins l'une des couches suivantes :
- une première couche extérieure antiadhésive, en contact avec le liquide contenu ;
- une seconde couche intermédiaire d'un matériau résistant à la pression du volume contenu dans le premier tube ; et
- une troisième couche intérieure à traitement ultraviolet ;
(c) des éléments de fermeture supérieure et inférieure (2103) de l'ensemble (2103) ; et
(d) des éléments de connexion inter-colonnes (2104), comprenant un connecteur de liquides entre les colonnes et une pluralité d'éléments de circulation de liquide auxiliaire ;
(e) des moyens de nettoyage comprenant au moins deux anneaux concentriques, un premier anneau extérieur et un second anneau intérieur et dans lesquels les deux sont joints au moyen d'une pluralité de rayons, et un élément périphérique qui nettoie par friction les parois avec lesquelles il entre en contact sans les endommager ;
(f) un élément de refroidissement et/ou de réfraction, consistant en un troisième tube extérieur par rapport au premier tube extérieur de telle sorte qu'il agisse comme un manchon, comprenant lui-même des sous-éléments de réfraction et/ou un liquide de refroidissement et/ou de réfraction ;
(g) une solution contenant des zéolites ;
de telle sorte que le second tube intérieur (2102) est inséré dans le premier tube extérieur (2101), l'ensemble étant fermé à ses extrémités, les éléments de connexion (2104) entre les colonnes étant situés à la fois en sa partie supérieure et sa partie inférieure ; et
(ii) des moyens d'alimentation et de génération de turbulences pour générer les turbulences d'un liquide à l'état gazeux et pour son alimentation, ledit liquide à l'état gazeux étant au moins l'un de ceux sélectionnés parmi :
a) de l'air ;
(b) du N₂ ;
(c) du CO₂ ;
(d) des gaz d'émission d'échappements industriels ; ou
(e) une combinaison de ceux-ci.

3. Procédé continu selon la revendication 1, **caractérisé en ce que** le champ magnétique d'excitation est continu.

4. Procédé continu selon la revendication 1, **caractérisé en ce que** le champ magnétique d'excitation est alternatif.

5. Procédé continu selon la revendication 1, **caractérisé en ce que** les moyens de pré-concentration (51) des seconds moyens de séparation (50) comprennent au moins l'un des systèmes suivants :
- un système de filtration de membrane immergé (MBR) ;
- un système de filtration tangentiel ;
- des moyens de floculation et/ou de coagulation ;
- des moyens d'électrocoagulation ;
- un système à ultrasons ;
- des moyens de recompression mécanique de vapeur (RMV) ;
- des moyens de centrifugation ;
- des moyens de décantation ; ou
- une combinaison des éléments ci-dessus ;
de telle sorte que 99% de l'eau présente dans la culture soient éliminés.

6. Procédé continu selon la revendication 1, **caractérisé en ce que** les moyens de concentration (52) comprennent au moins l'un des systèmes sélectionnés parmi les suivants :
- un système de recompression mécanique de vapeur (RMV) ;
- des moyens de décantation de l'élément pré-concentré ;
- des moyens de centrifugation ;
- des moyens de pression ; ou
- une combinaison des éléments ci-dessus ;
de telle sorte qu'un concentré d'humidité relative comprise entre 40% et 90%, de préférence entre 65 et 70%, soit obtenu.

7. Procédé continu selon la revendication 1, **caractérisé en ce que** les moyens de séchage (53) comprennent au moins :
(i) des moyens de séchage thermique pour un séchage par air chaud, de telle sorte que le produit concentré puisse être séché au moyen de l'insertion d'air chaud ayant une température non inférieure à 75°C ;
(ii) des moyens de lyophilisation ;
(iii) des évaporateurs multi-effets.

8. Procédé continu selon les revendications 1 et 2, **caractérisé en ce que** le CO₂ utilisé pour alimenter la culture de phytoplanctons provient d'une réaction des moyens de transformation.

9. Procédé continu selon une quelconque des revendications précédentes, **caractérisé en ce que** le système incorpore des générateurs de vent, de telle sorte que lesdits générateurs de vent génèrent l'électricité réelle pour l'utilisation du système, une accumulation excessive d'énergie étant générée dans la culture de phytoplanctons elle-même.

10. Procédé continu selon une quelconque des revendications précédentes, **caractérisé en ce que** le système est installé dans la mer, au moyen de flotteurs et partiellement immergé.

11. Procédé continu selon la revendication 1, **caractérisé en ce que** les moyens d'éclairage sont soit intérieurs soit extérieurs par rapport aux modules de production pour la production de culture et qu'ils comprennent des moyens d'éclairage naturel et/ou artificiel.

12. Procédé continu selon la revendication 1, dans lequel la culture de phytoplanctons est traitée à un rythme allant jusqu'à 20 000 litres/heure.

13. Procédé continu selon la revendication 12, dans lequel la culture de phytoplanctons est traitée à un rythme allant jusqu'à 5 000 litres/heure.
